# EUROPEAN PATENT APPLICATION

(11) **EP 4 397 302 A1**
(43) Date of publication of application: **10.07.2024**
(21) Application number: 22863565.2
(22) Date of filing: 01.09.2022
(51) Int. Cl.: A61K 9/08, A61K 9/10, A61K 9/107, A61K 31/575, A61K 31/56, A61K 47/26, A61K 47/10, A61K 47/38, A61K 47/36, A61K 47/58, A61P 27/12, A61P 27/02

(54) **OPHTHALMIC FORMULATION FOR PREVENTING AND/OR TREATING CATARACTS BY EYE DROP ADMINISTRATION**

(30) Priority: 03.09.2021 CN 202111033229
(71) Applicant: Chengdu Ruimu Biopharmaceuticals Co., Ltd., Chengdu, Sichuan 611135 (CN)
(72) Inventor: DONG, Qing, Chengdu, Sichuan 611135 (CN); XUE, Lubing, Chengdu, Sichuan 611135 (CN)
(74) Representative: Altmann Stößel Dick Patentanwälte PartG mbB
(86) International application number: PCT/CN2022/116432
(87) International publication number: WO 2023/030430

(57) **Abstract**

An ophthalmic formulation for preventing and treating cataracts by eye drop administration, which comprises an active substance for treating eye diseases and a pharmaceutically acceptable carrier or excipient. The active substance for treating eye diseases is an oxysterol, including lanosterol or 25-hydroxycholesterol; the pharmaceutically acceptable carrier or excipient include a surfactant, a thickening agent, a cosolvent, and a solvent; the content of the oxysterol in the formulation is 0.01-5 mg/mL; and the mass ratio of the surfactant, the thickening agent, the cosolvent, and the oxysterol is (1-300):(1-100):(100-3000):1, with the balance being the solvent. Upon eye drop administration, the formulation can allow the active substance to be selectively enriched in the lens of the test animals to accurately exert the effect of treating cataracts; moreover, the active substance is not distributed in the aqueous humor and the vitreous body, thereby avoiding toxic and side effects on other eye tissue and the whole body. The present invention solves the technique challenge in the field of ophthalmic drug delivery, that is desired to be solved by people for a long period of time, and has extremely high clinical application values and social values.

## Description

### Field of the invention

The present invention belongs to the field of ophthalmic medicaments, and specifically relates to an ophthalmic formulation for preventing and/or treating cataracts by eye drops.

### Background of the invention

Cataracts are caused by aging and other factors leading to the gradual aging and turbidity of the lens, blocking the entry of light into the eye, and thus producing visual impairment or even blindness. Cataracts are currently the most common eye diseases worldwide, which can cause blindness. According to a report by the World Health Organization (WHO), the blindness rate caused by cataracts is over 40%, while the proportion of people above 50 years old who become blind due to cataracts is about 47.8%. Moreover, the cataracts account for 90% of the blindness in developed countries. There are many patients with cataracts, and currently there is no reliable drug treatment method to replace surgery for preventing or treating cataracts. At present, surgical treatment is the only confirmed and effective method for treating cataracts (Jingjie Xu et al: Advances in pharmacotherapy of cataracts, Ann Transl Med 2020; 8(22): 1552).

In the process of exploring the occurrence and development of cataracts, researchers have found that oxysterols (oxidized cholesterols), including 25-hydroxycholesterol (25-HC, CAS#: 2140-46-7) and lanosterol (LAN, CAS#: 79-63-0), have the effect of reversing cataracts that have already occurred and restoring lens transparency (Ling Zhao et. al., Lanosterol reverses protein aggregation in cataracts, Nature 523, 607-611, 2015; Makley et. al., Pharmacological chaperone for α-crystallin partially restores transparency in cataract models, Science 350 (6261), 674-677, 2015).

The function of the lens is to transmit and concentrate light to the retina, and it is one of the refractive media of the eye. The crystallines in the lens are subdivided into three groups, namely α-, β-, and γ-crystallins, which are arranged in a regular manner, and the interactive structure between crystallin molecules is the basis for determining the transparency and refractive index of the lens. The pathological basis of cataracts is abnormal folding and aggregation of crystallins, which alters the interaction between crystallins, and reduces their fluidity and stability. The aggregated proteins produce cloudy and opaque lenses, which prevent light from entering the eyes and lead to cataracts.

Lanosterol and 25-hydroxycholesterol can prevent the polymerization of crystallins, improve lens morphology, and restore transparency. Gestwicki et al. reported that oxysterol, including 25-hydroxycholesterol, can enhance the protective activity of α-crystallin and clear cataracts. Kang Zhang's team reported that lanosterol binds to β- and γ-crystallins, that can prevent crystallin polymerization and clear cataracts (Ling Zhao et. al., Nature 2015; Makley et. al., Science 2015).

LAN is a key cyclization intermediate for the biosynthesis pathway of cholesterol in the body, and synthesized by catalysis of lanosterol synthase (LSS); 25-hydroxycholesterol is synthesized from cholesterol catalyzed by 25-hydroxycholesterase in the body. In pathological conditions, the concentration of these metabolic intermediates in the lens is insufficient to effectively reverse cataracts, and the active substances need to be supplemented. Oral supplementation and other methods are the most common approach, but by systemic supplementation, these active substances are mostly involved in the biochemical processes throughout the body, and the intermediates reaching the lens are still not sufficient to have effective concentrations. In addition, due to the broad physiological activity of cholesterols, introducing exogenous oxysterols by systemic administration may result in unpredictable pathological and physiological reactions. For example, lanosterol is a key intermediate in the biosynthesis of cholesterol in the body, and 25-hydroxycholesterol is an oxidation product of cholesterol, closely related to inflammation or infection. It is also a liver X receptor (LXR) agonist, and ingestion of LXR agonists may lead to liver fat synthesis and hypertriglyceridemia (Willinger, et al., Oxysterols in intestinal immunity and inflammation, Journal of Internal Medicine, 2019, 285, 367-380; Donovan Duc, et al., Oxysterols in Autoimmunity, Int. J. Mol. Sci. 2019, 20, 4522, 1-16; Cystger, et al., Nat. Rev. Immunol. 14(11), 731-743 (2014); Tong Wu, Hong-Jun Du, Research progress of liver X receptors in ophthalmic diseases, new advances in ophthalmology, Recent Advances in Ophthalmology, 39(9), 886-897, 2019).

Therefore, local delivery into the eye is the best way to prevent and treat cataracts with this type of active substances.

However, the surface of the cornea in the anterior part of the eye is covered by the tear film, and the cornea itself is composed of a lipid layer, a watery layer, and a mucin layer. From outside to inside, drug molecules need to penetrate into the aqueous stroma layer of the cornea, and then pass through the lipid layer in order to enter the anterior chamber and reach the lens through the pupil. To achieve the goal of preventing and treating cataracts by allowing drugs to penetrate natural barriers involving tissue anatomy, physiology, and biochemistry, it is necessary to overcome the technical difficulties of prior eye drops (Thrimawithana, T.R. et al., Drug delivery to the lens for the management of cataracts, Advanced Drug Delivery Reviews (2018), 126, 185-194).

So far, to overcome the corneal barrier and deliver oxysterols to the lens, ocular injection is commonly attempted. For example, researchers injected nanoparticles prepared from lanosterol, polycaprolactone, lecithin, and DSPE-PEG-COOH into the vitreous cavity of an animal cataract model (once every 3 days), combined with eye drops of lanosterol solution at a concentration of 25 mM (15% cyclodextrin and 18% ethanol), six times a week, for 6 weeks, and the cataract disappeared; however, administration by eye drops to the experimental animals alone cannot treat cataracts (Ling Zhao et al., Nature, 2015; Kang Zhang and Shenyang Hou, US2017/0065617 A1). That demonstrates that in prior art, invasive methods such as injection are necessary to overcome the ocular barrier, while direct eye drops of lanosterol solution cannot allow drugs to smoothly pass through the corneal and lens barriers and enter into the lens to play therapeutic effects.

However, active substances in the aqueous humor and/or vitreous body still need to permeate through the lens capsule to enter the lens and play their effects. Many studies in the prior art have found that even if the active substance oxysterol is directly injected into the vitreous body, it is still difficult for it to enter the lens and produce therapeutic effects due to the barrier of the lens capsule.

For example, the researchers injected the lanosterol/thermogel formulation prepared by poly-(DL-lactic acid-co-glycolic acid)-poly(ethylene glycol)-poly-(DL-lactic acid-co-glycolic acid) (PLGA-PEG-PLGA) into the vitreous cavity of experimental rabbits (at a concentration of 400 mg/g), and the intravitreous LAN concentration could be maintained at >50 ng/mL for 3 weeks (Lei Lv et al., Quantitation of lanosterol in the vitreous humor of rabbits after ocular administration of lanosterol/thermogel formulation by ultra high performance liquid chromatography-tandem mass spectrometry with the electrospray ionization mode, J Chromatogr. A, 2017; 1519: 83-90). Nagai et al. injected lanosterol nanoparticle injection (at a concentration of 2 mg/mL, once every 2 days) into the vitreous cavity of experimental rats for 6 weeks, without blocking the process of lens opacity (Noriaki Nagai, et al: The Intravitreal Injection of Lanosterol Nanoparticles Rescues Lens Structure Collapse at an Early Stage in Shumiya Cataract Rats, Int. J. Mol. Sci. 2020, 21, 1048).

In addition, *in vitro* experiments have further confirmed that oxysterols are difficult to break through the barrier of the lens and enter the lens to produce therapeutic effects. For example, Shanmugam et al. soaked the cataract nucleus obtained after cataract extraction in a lanosterol solution at a concentration of 25 mM for 6 days, and there was no significant improvement in cataract clarity (Shanmugam et al., Effect of lanosterol on human cataract nucleus, Indian J Ophthalmol. 63(12): 888-890, 2015); Daszynski et al. soaked rat lenses in a LAN liposome solution (15 mM) for 48 h, or soaked the lens fragments of cataract patients in lanosterol (0.20 mM lanosterol solution) or 25-hydroxycholesterol buffer (concentrations: 0.25 mM and 0.50 mM, 37 °C, 72 h), but did not find that lanosterol or 25-hydroxycholesterol can bind to crystallins to solve cataract problems (Daszynski, et. al., Failure of oxysterols such as Lanosterol to Restore Lens Clarity from Cataracts, Scientific Reports (2019) 9: 8459, 14 pages)

Based on the above, it has been shown that even if invasive vitreous injection is used to penetrate the corneal barrier, the medicament can only achieve higher concentrations in ocular fundus such as the retina, choroid, and vitreous body, but still cannot effectively enter the lens and play its effects. If intraocular lens injection is directly used, it may induce traumatic or posterior cataracts, which cannot be used in clinical practice.

In summary, oxysterols can effectively prevent and treat cataracts in the lens, but currently, there is no clinically feasible delivery method in ophthalmology to safely transport them into the lens. Moreover, due to their wide range of physiological activities, it is necessary to avoid the entry of exogenous oxysterols into the human body. The prior oral, injection, and implantation systems cannot guarantee the safety of such drugs. The use of non-invasive eye drops for drug delivery, different from the current delivery mechanisms of eye drops, can ensure their therapeutic concentration in the lens as an ideal mode of administration. Therefore, inventing ophthalmic formulations that can safely and effectively send drugs to the lens by eye drops is an urgent problem that needs to be solved in the field of ophthalmic formulations.

However, in addition to the difficulties in the drug delivery processes mentioned above, there are also solubility issues in preparing formulations with oxysterols as the active substances. Potential active substances such as oxysterols (e.g. lanosterol), as tetracyclic triterpenoid compounds, are soluble in chloroform, ethanol, diethyl ether, n-propanol, isopropanol, *N*,*N*-dimethylformamide (DMF), and dimethyl sulfoxide (DMSO), but have low solubility in water (Li and Forciniti, J. Chem. Eng. Data, 2020, 65, 2, 436-445). Therefore, organic solvents are commonly used to improve solubility, but their excessive uses can cause eye irritation, leading to potential safety issues. In addition to organic solvents, researchers have also used cyclodextrin or its derivatives as excipients (cyclodextrins, CYD, including α-cyclodextrin, β-cyclodextrin, hydroxypropyl-β-cyclodextrin), and by inclusion technology to disperse oxysterol molecules into cyclodextrin holes, a solution is prepared for animal research (Gu-Yin En, CN108472303 A, 2016; J.D. Sciamanna, US2020/0360403A1). But, the CYD inclusion complex cannot penetrate the biofilm, in which β-cyclodextrin and cholesterol may form a complex that is insoluble in the body and accumulates in the kidney, leading to serious nephrotoxicity (R.C. Rowe, P.J. Shersky, P.J. Weiler, Handbook of Pharmaceutical Excipients [M]. Beijing: Chemical Industry Press, 2005) and limiting its further development and application. Currently, only dog products are sold on the Internet (trade name: Lanomax^{®}).

Therefore, investigation on an ophthalmic formulation that can overcome the problem of drug solubility and pass through the corneal and lens barriers, to treat cataracts by eye drops, will offer significant clinical application and social benefits.

### Summary of the invention

The object of the present invention is to provide an ophthalmic formulation capable of delivering active substances for treating eye diseases, such as oxysterols, to the lens by eye drop administration for the treatment and prevention of cataracts.

The present invention provides an ophthalmic formulation for eye drop administration, which is composed of active substances for treating eye diseases and pharmaceutically acceptable carriers or excipients;
the active substances for treating eye diseases are oxysterols, including lanosterol or 25-hydroxycholesterol;
the pharmaceutically acceptable carriers or excipients contain surfactants, thickening agents (tackifiers), cosolvents, and a solvent;
the content of oxysterols in the formulation is 0.01-5 mg/mL; and the mass ratio of the surfactant, the thickening agent, the cosolvent, and the oxysterol is (1-300):(1-100):(100-3000):1, with the balance being the solvent.

Further, the content of oxysterols in the formulation is 0.01-2 mg/mL.

Further, the content of oxysterols is 0.05-0.5 mg/mL.

Further, the content of oxysterols in the formulation is: 0.01 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.65 mg/mL, 0.7 mg/mL, 0.75 mg/mL, 0.8 mg/mL, 0.85 mg/mL, 0.9 mg/mL, 0.95 mg/mL, 1 mg/mL, 1.5 mg/mL or 2 mg/mL, 2.5 mg/mL, 3 mg/mL, 3.5 mg/mL, 4 mg/mL, 4.5 mg/mL or 5 mg/mL.

Further, the mass ratio of the surfactant, the thickening agent, the cosolvent, and the oxysterol is (6.7-250):(11-50):(100-2500):1, preferably (25-200):(11-48):(200-2500):1, and more preferably 25:12:(200-600): 1.

Further, the surfactants are non-ionic surfactants.

More further, the non-ionic surfactants are polysorbate, poloxamer, or alkyl polyglucoside (APG).

Further, the thickening agent is a combination of at least two of the following polymer compounds, including hydroxypropyl methylcellulose, hydroxypropyl cellulose, methyl cellulose, povidone, carbomer, polyethylene glycol, poloxamer, polyvinyl alcohol, hydroxyethyl cellulose, xanthan gum, hyaluronic acid or its salt, alginic acid or its salt, carboxymethyl cellulose or its salt.

More further, the thickening agent is a combination of two polymer compounds mentioned above, in which the mass ratio of two polymer compounds is 1:(0.1-10), and preferably 1:(0.6-5).

More further, the mass ratio of two polymer compounds is 1:1.

More further, the thickening agent is a combination of povidone and hydroxypropyl cellulose, wherein the mass ratio of hydroxypropyl cellulose to povidone is 1:(1-2), and preferably 1:(1-1.2);
alternatively, the thickening agent is a combination of povidone and hydroxypropyl methylcellulose, wherein the mass ratio of povidone to hydroxypropyl methylcellulose is 1:(1-3), and preferably 1:(1-1.5);
alternatively, the thickening agent is a combination of povidone and carbomer, wherein the mass ratio of povidone to carbomer is 1:(0.5-2), and preferably 1:1;
alternatively, the thickening agent is a combination of povidone and polyethylene glycol, wherein the mass ratio of polyethylene glycol to povidone is 1:(1-8), and preferably 1:5.

More further, the oxysterol is 25-hydroxycholesterol, and the thickening agent is a combination of at least two of the following polymer compounds, including hydroxypropyl methylcellulose, hydroxypropyl cellulose, methyl cellulose, povidone, carbomer, polyethylene glycol, poloxamer, polyvinyl alcohol, hydroxyethyl cellulose, xanthan gum, hyaluronic acid or its salt, alginic acid or its salt, carboxymethyl cellulose or its salt.

More further, the oxysterol is 25-hydroxycholesterol, and the thickening agent is a combination of povidone and hydroxypropyl cellulose, wherein the mass ratio of hydroxypropyl cellulose to povidone is 1:(1-2), and preferably 1:(1-1.2);
alternatively, the thickening agent is a combination of povidone and hydroxypropyl methylcellulose, wherein the mass ratio of povidone to hydroxypropyl methylcellulose is 1:(1-3), and preferably 1:(1-1.5);

More further, the oxysterol is lanosterol, and the thickening agent is a combination of at least two of the following polymer compounds, including hydroxypropyl methylcellulose, hydroxypropyl cellulose, methyl cellulose, povidone, carbomer, polyethylene glycol, poloxamer, polyvinyl alcohol, and hydroxyethyl cellulose.

More further, the oxysterol is lanosterol, and the thickening agent is a combination of povidone and hydroxypropyl cellulose, wherein the mass ratio of hydroxypropyl cellulose to povidone is 1:(1-2), and preferably 1:(1-1.2);
alternatively, the thickening agent is a combination of povidone and hydroxypropyl methylcellulose, wherein the mass ratio of povidone to hydroxypropyl methylcellulose is 1:(1-3), and preferably 1:(1-1.5);
alternatively, the thickening agent is a combination of povidone and carbomer, wherein the mass ratio of povidone to carbomer is 1:(0.5-2), and preferably 1:1;
alternatively, the thickening agent is a combination of povidone and polyethylene glycol, wherein the mass ratio of polyethylene glycol to povidone is 1:(1-8), and preferably 1:5.

Further, the solvent in the pharmaceutically acceptable carriers or excipients is a polar solvent, and preferably water.

Further, the co-solvents in the pharmaceutically acceptable carriers or excipients are selected from at least one of liquid polyethylene glycol, propylene glycol, glycerol, polyoxyethylene hydrogenated castor oil, or castor oil polyoxyethylene ether, and preferably liquid polyethylene glycol.

Further, the formulations comprise the following components:
an active substance for treating eye diseases: lanosterol, at a content of 0.01-0.2 mg/mL;
a surfactant: polysorbate or poloxamer, at a content of 6.7-250 times that of lanosterol;
a thickening agent: its content is 11-50 times that of lanosterol, and the thickening agent is a combination of povidone and hydroxypropyl cellulose, wherein the mass ratio of hydroxypropyl cellulose to povidone is 1:(1-1.2); alternatively, the thickening agent is a combination of povidone and hydroxypropyl methylcellulose, wherein the mass ratio of povidone to hydroxypropyl methylcellulose is 1:(1-1.5); alternatively, the thickening agent is a combination of povidone and carbomer, wherein the mass ratio of povidone to carbomer is 1:1; alternatively, the thickening agent is a combination of povidone and polyethylene glycol, wherein the mass ratio of polyethylene glycol to povidone is 1:5;
co-solvent: liquid polyethylene glycol, propylene glycol, glycerol, polyoxyethylene castor oil or castor oil polyoxyethylene ether, at a content of 100-2500 times that of lanosterol; the solvent is water.

Alternatively, the formulations comprise the following components:
an active substance for treating eye diseases: 25-hydroxycholesterol, at a content of 0.1 mg/mL;
a surfactant: polysorbate, at a content of 25-250 times that of 25-hydroxycholesterol;
a thickening agent: its content is 12 times that of 25-hydroxycholesterol, and the thickening agent is a combination of povidone and hydroxypropyl cellulose, wherein the mass ratio of hydroxypropyl cellulose to povidone is 1:1; alternatively, the thickening agent is a combination of povidone and hydroxypropyl methylcellulose, wherein the mass ratio of povidone to hydroxypropyl methylcellulose is 1: 1;
co-solvent: liquid polyethylene glycol or glycerol, at a content of 100-1750 times that of 25-hydroxycholesterol; the solvent is water.

Further, the pharmaceutically acceptable carriers or excipients in the formulations also comprise any one or more of osmotic pressure regulators, pH regulators, or preservatives;
the osmotic pressure regulators are any one or more of glucose, sodium chloride, potassium chloride, mannitol, sorbitol, sodium citrate, potassium citrate, and glycerol;
the pH regulators are any one or more of hydrochloric acid, sodium hydroxide, acetic acid or its salt, citric acid or its salt, fumaric acid, succinic acid, sorbic acid, phosphoric acid, sodium dihydrogen phosphate, disodium hydrogen phosphate, boric acid, borax, tartaric acid or its salt;
the preservatives are any one or more of sorbic acid, chlorobutanol, sodium chlorite, sodium perborate, quaternary ammonium salts (including benzalkonium chloride, benzalkonium bromide, polyquaternium-1, hexadecyltrimethylammonium bromide), parabens (including methyl hydroxybenzoate, ethyl hydroxybenzoate, propyl hydroxybenzoate), and phenylmercuric nitrate; preferably, the quaternary ammonium salts include benzalkonium chloride, benzalkonium bromide, polyquaternium-1 and/or hexadecyltrimethylammonium bromide, and the parabens include methyl hydroxybenzoate, ethyl hydroxybenzoate, and/or propyl hydroxybenzoate.

Further, the ophthalmic formulations contain nanoparticle structures, which are self-assembled from the carrier or excipient components of the ophthalmic formulation; the nanoparticles comprise active substances for treating eye diseases.

More further, the nanoparticles are spherical, with a particle size of 5-900 nm, preferably 5-50 nm and/or 200-700 nm.

The present invention also provides a method for preparing the formulations, which comprises the following steps:
(1) A surfactant and a thickening agent are added to the solvent, and then mixed to obtain a mixed solution;
(2) An active substance for treating eye diseases is added to the mixed solution obtained in step (1), to which a co-solvent is added or not added, and then dispersed and mixed to obtain the initial suspension;
(3) The initial suspension obtained in step (2) is stirred and dispersed and/or homogenized to obtain the formulations.

Further, the dispersion in step (2) is selected from at least one of mechanical stirring and dispersion, magnetic stirring and dispersion, vortex vibration and dispersion, shear dispersion, homogeneous dispersion, grinding and dispersion, and ultrasonic dispersion.

The present invention also provides the use of the formulations in the manufacturer of medicaments for the prevention and treatment of lens diseases in humans or animals.

Further, the medicaments are that for preventing and treating cataracts, and preferably that reducing crystallin aggregation and lens opacity.

Further, the medicaments are pharmaceutical formulations for ocular administration, and preferably that for ocular topical administration.

Oxysterols, including 25-hydroxycholesterol (25-HC) and lanosterol (LAN), can interact with crystallins in the lens to prevent crystallin aggregation, improve lens morphology and restore transparency. They have the effect of reversing cataracts and restoring lens transparency, so as to treat and prevent cataracts. By oral, intramuscular, and intravenous administration, oxysterols participate in the biochemical processes throughout the body and cannot reach effective concentrations in the lens; LAN and 25-HC injected into the vitreous body cannot penetrate the capsule and enter the lens; lens injection can cause traumatic or posterior cataracts.

The route of eye drop administration is a safe and convenient way, but to treat fundus diseases, medicaments need to penetrate the eye barrier and be delivered safely and effectively to the site of fundus lesions.

The present invention follows the optimization design principle of therapeutic effect/risk ratio, aiming to reducing the concentration of the formulation while achieving therapeutic effect, to minimize the risk. Due to mechanical actions such as blinking, only about 10% of eye drops remain on the surface of the eye, but most of them are discharged from the lacrimal ducts along with the tears, while a small portion enters the nasal cavity through the nasolacrimal ducts and even enters the bloodstream by absorption. The lower the concentration of the formulation, the smaller the risk borne by the tissues and organs, and the fewer the systemic toxic side effects produced. The concentration of lanosterol in the formulation of the present invention (not exceeding 4.68 mM) is lower than the concentration claimed by other patents, which can minimize toxic side effects as much as possible. At the same time, 20 µL of the formulation was dropped into the eyelid conjunctival sac at a concentration of 0.1 µg/µL, with approximately 0.2 µg remaining on the ocular surface. If calculated based on a rat lens volume of 0.03 cm³ (diameter = 3.87 mm), the LAN concentration in the lens after eye drops can reach 2.3-2.5 times the original content, with a penetration rate of 44-52%. Taking rabbits as an example, 50 µL of the formulation according to the present invention at a concentration of 0.11 µg/µL is dropped, and then calculated based on 10% staying on the ocular surface and entering the rabbit lens (diameter = 7.9 mm, volume = 0.258 cm³), the amount of lanosterol entering the lens can reach 3.6 times the original concentration, and the penetration rate can almost reach 100% (A.B. Weir and M. Collins (eds.), Assessing Ocular Toxicology in Laboratory Animals, 1 Molecular and Integrative Toxicology, DOI 10.1007/978-1-62703-164-6_1, © Springer Science+Business Media, LLC 2013; Pei-Xue Ling, Editor in Chief, Ophthalmic Drugs and Preparations, China Light Industry Press, 2010, P6). It has been shown that although the concentration of oxysterols used in the present invention is low, the utilization rate is very high, which can not only reduce the potential risk of systemic toxic side effects, but also fully ensure the dosage and efficacy of the medicament.

Another feature of the formulations according to the present invention is the formula advantages. The treatment of cataracts needs a long-term medication, and it is particularly important to avoid active excipients in the formula. For example, a low concentration formula with anti-infective activity may cause drug resistance in the ocular surface and nasolacrimal duct microbiota, and even induce infection. On the one hand, the concentration of the present invention is low, and on the other hand, the formulations do not require the use of preservatives, and for long-term using may have no potential complications.

According to literature reports, oxysterols have poor water solubility. In order to prepare oxysterol injections or eye drops, solvents such as ethanol and DMSO are commonly used. Alcohol solvents have irritating effects on the eyes, and thus their amounts must be controlled. Using DMSO as a co-solvent may increase the solubility of active substances, but it may allow more active substances to enter the systemic circulation. For example, injecting a 0.5% LAN solution, prepared with 15% DMSO, 20% ethanol, or a solution containing polysorbate 80, into the vitreous body of rats not only causes vitreous turbidness, but also poses a risk of promoting lens opacity.

The highly ordered and tightly arranged crystallins are rather transparent, and injection can cause the structural damage leading to traumatic cataracts. Therefore, it is difficult to deliver LAN to the lens by injection route (Gu-In En, CN108472303 A, 2016; Qin-Yuan Zheng, Jiapaer Zeyidan, 201610720166.X; Noriaki Nagai, et al: The Intravitreal Injection of Lanosterol Nanoparticles Rescues Lens Structure Collapse at an Early Stage in Shumiya Cataract Rats, Int. J. Mol. Sci. 2020, 21, 1048).

The inventors have performed extensive experiments and invented the combined use of non-ionic surfactants as solubilizing agents, or/and thickening agents, or/and co-solvents, together with the processing by physically stirring and mixing, high-speed shear dispersion, and high-pressure homogenization techniques. If necessary, heating and ultrasound can be used to prepare a solution of oxysterol LAN or 25-HC with water as the main vehicle, and the prepared solution has good stability. The prepared aqueous solution containing the oxysterols was detected by a laser particle analyzer, and its main particle size distribution was between 5-50 nm and/or 200-700 nm. Spherical particles can be observed under an electron microscope. The water-soluble solvent prepared in the present invention is soluble in tears; subsequently, oxysterol enters the lens through the cornea and lens capsule. In the lens absorption test of live animal by eye drops administration, for the aqueous eye drops of oxysterol prepared in the present invention in which water is used as the main vehicle, after eye drops were given to experimental animals, a significant increase in the concentration in the lens was detected, while the content of oxysterols was very low (rat test) or not detected (rabbit test) in the aqueous humor and vitreous body.

In the experiment performed with a cataract model induced by subcutaneous injection of sodium selenite solution in rats, the inventors have observed that the eye drops prepared in the present invention can delay the occurrence and development of cataracts in test animals. An elderly dog (15 years old) with cataracts can be observed a significant reduction in the opacity of the lens after administration of the LAN eye drops for 20 days, which was prepared in the present invention.

In the present invention, it has been proven by experiments that after eye drops topical administration of the ophthalmic formulations according to the present invention, the active substances can be efficiently and accurately delivered into the lens of test animals, and as a companion of crystallins, they can prevent the crystallins aggregation, clear cataracts, and play therapeutic and preventive effects on cataracts.

In animal experiments, it was unexpectedly discovered that the oxysterol eye drops prepared in the present invention have the advantages of safety and effectiveness. In particular, it was unexpectedly discovered that after eye drops in test animals, oxysterols were enriched in the lens; (the absorption experiment of eye drops in New Zealand rabbits has shown that LAN is enriched in the lens), while the concentration in aqueous humor and vitreous body was very low, implying the eye drops of the present invention have high selectivity for the target tissue.

The eye drops of the present invention can selectively and efficiently deliver active substances of oxysterols to the lens by eye drops, supplementing cataracts caused by the deficiency of oxysterols; that can also avoid unpredictable pathological and physiological reactions caused by cholesterols with broad physiological activities in the body.

Oxysterols in the lens can prevent the crystallins aggregation and clear cataracts; However, oxysterols can cause cardiovascular complications in the body, which are harmful to the body. So far, there is no technology in the pharmaceutical field able to deliver medicaments, including oxysterols, to the lens in a non-invasive manner. The novel low-concentration eye drops of the present invention has realized targeted delivery of medicaments into the lens. The most obvious clinical advantage is the delivery of oxysterols into the lens by eye drops, achieving a low content of active substances in the cataract eye drops of the present invention, while the bioavailability after eye drops is very high, effectively increasing the concentration of oxysterols in the lens for the treatment of cataracts. Due to the low concentration and high local bioavailability in the eyes, the eye drops greatly reduce the possibility of medicaments entering the body and producing toxic side effects through the conjunctiva and nasal cavity.

The active substance for treating eye diseases, as used in the present invention, means an active substance for treating eye diseases in humans or animals, that are present or not present in the body.

The nanoparticle, as used in the present invention, refers to a nanoscale spherical aggregate formed by the self-assembly of components of drug carriers or excipients in a solvent.

The solvents, as used in the present invention, means a liquid that can dissolve the components of drug carriers or excipients.

The surfactants, as used in the present invention, means the substances which can significantly reduce the surface tension of liquids; the non-ionic surfactants, as used in the present invention, refers to surfactants which do not dissociate in water.

The eye drop administration, as used in the present invention, means a method of administering a drug solution into the eye, belonging to the mucosal pathway.

The liquid polyethylene glycol (PEG), as used in the present invention, means a polyethylene glycol that is liquid at room temperature and atmospheric pressure, and preferably a polyethylene glycol with a weight-average molecular weight of not more than 1000.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. A transmission electron microscopy (TEM) photo of the sample prepared in Example 3.
Figure 2. A transmission electron microscopy (TEM) photo of the sample prepared in Example 3 after staining with dye.
Figure 3. Photos of lens opacity in the dog with cataract before and after using the eye drops of the present invention.

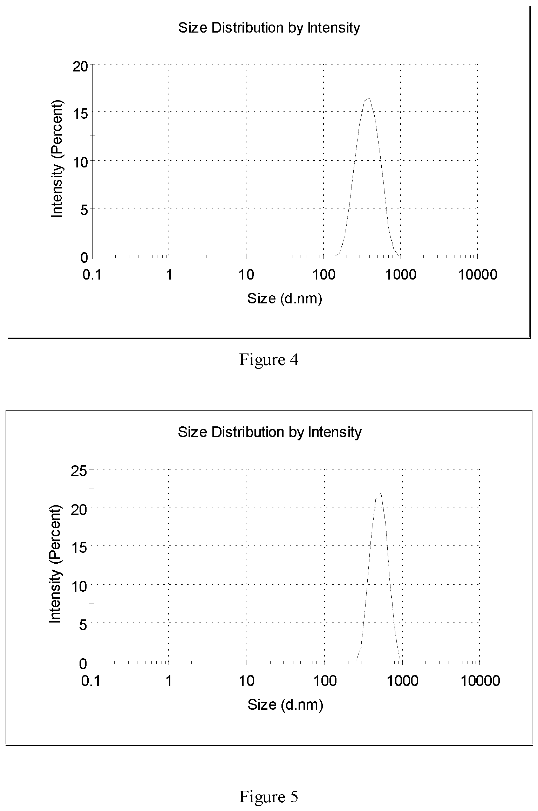

Figure 4. Particle size distribution of the sample (day 0) prepared in Example 5.
Figure 5. Particle size distribution of the sample (day 15, at room temperature) prepared in Example 5.

### Examples

The reagents or instruments used in the present invention can be obtained by purchasing commercially available products, and if specific conditions are not indicated, they shall be used according to conventional conditions or manufacturer's recommended conditions. Lanosterol (CAS: 79-63-0), content: 99.9% (HPLC, Chengdu Gelipu Biotech Co., Ltd., Chengdu); 25-hydroxycholesterol (CAS: 2140-46-7, content ≥98%, Shanghai Macklin Biochemical Technology Co., Ltd., Shanghai).

Some instruments and equipment are as follows:
ES225SM-DR(E) electronic analytical balance, Precisa (Switzerland);
DF-101S Heat-collection type thermostatic magnetic agitator, Gongyi Yingyu Rivet Factory (Henan, China);
WH-2 Mini Vortex meter, Shanghai Huxi Analysis Instrument Factory (Shanghai, China);
Disperse machine: T25 easy clean digital, IKA (Germany);
KQ-500 type ultrasonic cleaning machine, Kunshan Ultrasonic Instruments Co., Ltd (Kunshan, China)
AH-NANO Plus High Pressure Homogenizer, Antos Nano Technology (Suzhou) Co., Ltd. (China);
Mettler Toledo FE20 pH meter, Mettler-Toledo (Switzerland);
NS-90 Nanoparticle Size Analyzer, Zhuhai Omec Instrument Co., Ltd. (Zhuhai, China);
Agilent 1100 HPLC, Agilent Technologies Inc. (USA);
API4000 Triple Quadrupole Mass Spectrometer (Applied Biosystems, USA);
STY-1A Osmotic Pressure Measuring Instrument, Tiandatianfa Technology Corp., Ltd. (Tianjin, China).
Zetasizer Nano ZS, Particle Size and Zeta Potential Analyzer, Malvern (UK).

The methods for detecting the properties of the formulations according to the present invention were as follows:
Method for measuring the particle size:
   1 mL of sample prepared in the Example or Comparative Example was transferred to the sample cell. The detection temperature was set to 40 °C, and then the sample cell was put into the NS-90 Nanoparticle Size Analyzer to start the detection. Each sample was tested three times, and the particle size (in terms of light intensity distribution and % percentage) and polydispersity index (PdI) were represented with the average of three test results.
Method for measuring osmotic pressure:
   The molar concentration of osmotic pressure was measured by measuring the freezing-point depression of the solution. Procedures: the probe of STY-1A osmotic pressure measuring instrument was cleaned. Three portions of 100 µL distilled water were respectively added to three sample tubes, and after preheating the instrument, the sample tube containing 100 µL of distilled water was screwed onto the instrument probe, followed by selecting "clean 3 times" and clicking "Clean", that was repeated three times. Measuring: After filling in the sample information in the instrument information table, click "Testing"; a pipette was used to transfer 100 µL of sample into the sample tube, which was gently screwed onto the instrument, followed by clicking "Start" for testing. The test was repeated three times, and the average of the three test results was taken as the result. In actual animal experiments, if the osmotic pressure does not reach isotonic level, the aforementioned osmotic pressure regulator was used to adjust to achieve or approach isotonic level.
Method for measuring pH value:
   The FE20 pH meter was calibrated using pH buffer solutions (pH 4.00, 6.86, and 9.18, respectively). The electrodes were rinsed with pure water, excess water was sucked off with fiber-free paper, and then the electrodes were immersed in the liquid sample to be tested, followed by pressing the reading key to start the measuring. After the reading stabilized, the data obtained was the pH value of the sample.

If the pH value of the test solution was <5 or >9, the solution needed to be adjusted to pH 6-8 with acid or alkali. The commonly used pH regulators were NaOH and HCl, phosphoric acid and phosphate (e.g. sodium dihydrogen phosphate, disodium hydrogen phosphate), citric acid and citrate (e.g. sodium citrate), boric acid and borax; in actual animal experiments, if the pH value of the detected solution did not meet the requirements of ophthalmic formulations, the above pH regulator was used to adjust it.

### Example 1. Preparing the ophthalmic formulation of the present invention

The materials and proportions used are shown in Table 1. The preparation procedures: first, polysorbate 80, PVP K12, HPMC and liquid PEG (PEG400) were weighed separately and put into a 100 mL polypropylene centrifuge tube, to which was added a suitable amount of water for injection, and then the solution was stirred for 30 min. After that, 6.0 mg of lanosterol was added, and subsequently, water for injection was added up to 60 mL. The resultant solution was stirred and mixed for additional 10 min to get the mixed solution, which was dispersed with a disperse machine at a speed of 12000-15000 rpm for 3 min, and after shutdown and disappearance of the foam, the dispersion solution was transferred to the high-pressure homogenizer, in which the temperature was controlled to be at 5±5 °C, and homogenized at the pressure of 400 Bar for 2 min. Then, the pressure was increased to 1200-1400 Bar and homogenized for 20 min, and subsequently the pressure was reduced to 500 Bar and homogenized for 2 min before discharging. Clear homogeneous solution was obtained after foam disappeared naturally. The pH value and osmotic pressure were measured, and then sodium citrate (0.10 g) and sodium chloride (0.4 g) were added. The resultant solution was adjusted to pH 7.0 with 0.1N HCl or 0.1N NaOH, with an osmotic pressure of 302 mOsmol/kg. The solution was filtered through a filter membrane under reduced pressure to obtain the product as a solution.
Determining the concentration of the product by HPLC:
Detection instrument: Agilent 1100 high-performance liquid chromatography;
Chromatographic conditions: chromatographic column, Agilent ZORBAX Eclipse Plus C18, 4.6 × 100 mm, 3.5 µm; flow rate 1.0 mL/min; detection wavelength 205 nm; mobile phase: MeOH (60%)-acetonitrile (40%) isocratic elution. The sample was diluted with the mobile phase in a ratio of 1:5, and then 10 µL of sample solution was injected into HPLC. The content was determined to be 0.098 mg/mL by HPLC.

The particle size was 20.6 nm (85.6%), and PdI was 0.266.

The test results of the particle size (main particle sizes and their distribution proportions): the particle size 67.7 nm (46.2%) and 75.8 nm (38.9%); PdI (Polydispersity Index): 0.276;

The average Zeta potential: -5.03 ± 4.33 mV (25 °C).

The product was stored at 40 °C in dark for 30 days, and the appearance was not obviously changed. The particle size was tested to be 40.68 nm (88.8%), with PdI 0.249; and the content was determined to be 0.093 mg/mL by HPLC.

The experimental results for eye drop absorption in animals: three rats (6 eyes) were chosen, and then the test solution was administered to both eyes of the rats at 20 µL/eye by eye drops. After 1.5 h, the animals were euthanized, and then the vitreous body and lens were quickly collected. The content of LAN was determined. Test results: lanosterol content, in the lens: 5.16±1.90 (µg/mL); in the vitreous body: 0.045±0.091 (µg/mL).

### Example 2. Preparing the ophthalmic formulation of the present invention

The materials and proportions used are shown in Table 1, and the preparation procedures were the same as that of Example 1, to obtain the solution;
The test results of the particle size: the particle size 441.8 nm (100.0%); PdI 0.190;
Determination of the content by HPLC:
   Detection instrument: Agilent 1100 high-performance liquid chromatography;
   Chromatographic conditions: chromatographic column, Agilent ZORBAX Eclipse Plus C18, 4.6 × 100 mm, 3.5 µm; flow rate 0.8 mL/min; detection wavelength 205 nm; mobile phase: MeOH (85%)-0.1%H₃PO₄(15%) isocratic elution. The sample was diluted with the mobile phase in a ratio of 1:5, and then 10 µL of sample solution was injected into HPLC. The content was determined to be 0.095 mg/mL by HPLC.

The product was stored at 40 °C in dark for 15 days, and the appearance was not obviously changed. The particle size was tested to be 413.2 nm (94.5%), PdI 0.214; and the content was determined as 0.090 mg/mL by HPLC.

### Example 3. Preparing the ophthalmic formulation of the present invention

The materials and proportions used are shown in Table 1, and the preparation procedures and the content determination were the same as that of Example 1, to obtain the solution;

The test results of the particle size: the particle size 416.3 nm (100.0%); PdI 0.214; the content by HPLC: 0.078 mg/mL.

The product was stored at 2-8 °C in dark for 30 days, and the appearance was not obviously changed. The particle size was tested to be 478.5 nm (98.2%), PdI 0.245; and the content was determined as 0.077 mg/mL by HPLC.

The experimental results for eye drop absorption in the lens of animals: two New Zealand rabbits (4 eyes) were used, and then the test solution was administered at 50 µL/eye by eye drops. After 1.5 h, the animals were euthanized, and then the aqueous humor, vitreous body and lens were quickly collected, in which the content of LAN was determined. Test results: no lanosterol was detected in the aqueous humor and vitreous body (below the detection limit, LCQ ≤ 0.002 µg/mL); the content of lanosterol in the lens was 2.72±0.16 (µg/mL); the content of lanosterol was detected to be 0.76 ± 0.01 (µg/mL) in the lens of a blank control animal (one New Zealand rabbit, two eyes, not administered).

### Example 4. Preparing the ophthalmic formulation of the present invention

The materials and proportions used are shown in Table 1, and the preparation procedures and the content determination were the same as that of Example 1, to obtain the solution;

The test results of the particle size: the particle size 531.6 nm (100.0%); PdI 0.165; the content by HPLC: 0.077 mg/mL.

The product was stored at room temperature in dark for 15 days, and the appearance was not obviously changed. The particle size was tested to be 935.1 nm (88.2%), PdI 0.340; and the content was determined as 0.074 mg/mL by HPLC.

### Example 5. Preparing the ophthalmic formulation of the present invention

The materials and proportions used are shown in Table 1, and the preparation procedures and the content determination were the same as that of Example 1, to obtain the solution;

The test results of the particle size: the particle size 401.3 nm (98.8%); PdI 0.221; the content by HPLC: 0.063 mg/mL.

The product was stored at room temperature in dark for 15 days, and the appearance was not obviously changed. The particle size was tested to be 613.2 nm (100.0%), PdI 0.350; and the content was determined as 0.064 mg/mL by HPLC.

The experimental results for eye drop absorption in lens of animals: three rats (6 eyes) were selected, and then the test solution was administered at 20 µL/eye by eye drops. After 1.5 h, the animals were euthanized, and then the lens was quickly collected, in which the content of LAN was determined. Test results: the content of LAN in the lens was 2.89±0.60 (µg/mL).

### Example 6. Preparing the ophthalmic formulation of the present invention

The materials and proportions used are shown in Table 1, and the preparation procedures and the content determination were the same as that of Example 1, to obtain the solution;

The test results of the particle size: the particle size 10.7 nm (99.1%); PdI 0.224; the content by HPLC: 0.070 mg/mL.

The product was stored at room temperature in dark for 15 days, and the appearance was not obviously changed. The particle size was tested to be 11.2 nm (91.7%), PdI 0.246; and the content was determined as 0.069 mg/mL by HPLC.

The experimental results for absorption in lens of animals: three rats (6 eyes) were used, and then the test solution was administered at 20 µL/eye by eye drops. After 1.5 h, the animals were euthanized, and then the lens was quickly collected, in which the content of LAN was determined. Test results: the content of LAN in the lens was 4.83±2.15 (µg/mL).

### Example 7. Preparing the ophthalmic formulation of the present invention

The materials and proportions used are shown in Table 1, and the preparation procedures and the content determination were the same as that of Example 1, to obtain the solution;

The test results of the particle size: the particle size 472.5 nm (99.4%); PdI 0.205; the content by HPLC: 0.072 mg/mL.

The product was stored at 2-8 °C in dark for 15 days, and the appearance was not obviously changed. The particle size was tested to be 513.2 nm (97.1%), PdI 0.235; and the content was determined as 0.070 mg/mL by HPLC.

### Example 8. Preparing the ophthalmic formulation of the present invention

The materials and proportions used are shown in Table 1, and the preparation procedures and the content determination were the same as that of Example 1, to obtain the solution;

The test results of the particle size: the particle size 508.5 nm (97.7%); PdI 0.245; the content by HPLC: 0.080 mg/mL.

The product was stored at 40 °C in dark for 15 days, and the appearance was not obviously changed. The particle size was tested to be 423.5 nm (90.1%), PdI 0.223; and the content was determined as 0.078 mg/mL by HPLC.

The experimental results for absorption in lens of animals: three rats (6 eyes) were used, and then the test solution was administered at 20 µL/eye by eye drops. After 1.5 h, the animals were euthanized, and then the lens was quickly collected, in which the content of LAN was determined. Test results: the content of LAN in the lens was 5.68±1.60 (µg/mL).

### Example 9. Preparing the ophthalmic formulation of the present invention

The materials and proportions used are shown in Table 1, and the preparation procedures and the content determination were the same as that of Example 1, to obtain the solution;

The test results of the particle size: the particle size 572.2 nm (85.3%); PdI 0.529; the content by HPLC: 0.085 mg/mL.

The product was stored at 2-8 °C in dark for 15 days, and the appearance was not obviously changed. The particle size was tested to be 583.8 nm (80.2%), PdI 0.545; and the content was determined as 0.083 mg/mL by HPLC.

The experimental results for absorption in lens of animals: three rats (6 eyes) were used, and then the test solution was administered at 20 µL/eye by eye drops. After 1.5 h, the animals were euthanized, and then the lens was quickly collected, in which the content of LAN was determined. Test results: the content of LAN in the lens was 4.01±1.65 (µg/mL).

### Example 10. Preparing the ophthalmic formulation of the present invention

The materials and proportions used are shown in Table 1, and the preparation procedures and the content determination were the same as that of Example 1, to obtain the solution;

The test results of the particle size: the particle size 12.8 nm (99.0%); PdI 0.175; the content by HPLC: 0.073 mg/mL.

The product was stored at 40 °C in dark for 15 days, and the appearance was not obviously changed. The particle size was tested to be 13.2 nm (91.5%), PdI 0.222; and the content was determined as 0.072 mg/mL by HPLC.

### Example 11. Preparing the ophthalmic formulation of the present invention

The materials and proportions used are shown in Table 1, and the preparation procedures were the same as that of Example 1, while the content determination were the same as that of Example 2, to obtain the solution;

The test results of the particle size: the particle size 455.6 nm (99.7%); PdI 0.249; the content by HPLC: 0.051 mg/mL.

The product was stored at 40 °C in dark for 15 days, and the appearance was not obviously changed. The particle size was tested to be 216.8 nm (95.6%), PdI 0.351; and the content was determined as 0.049 mg/mL by HPLC.

### Example 12. Preparing the ophthalmic formulation of the present invention

The materials and proportions used are shown in Table 1, and the preparation procedures and the content determination were the same as that of Example 1, to obtain the solution;

The test results of the particle size: the particle size 420.0 nm (100.0%); PdI 0.205; the content by HPLC: 0.037 mg/mL.

The product was stored at 40 °C in dark for 30 days, and the appearance was not obviously changed. The particle size was tested to be 452.0 nm (94.0%), PdI 0.256; and the content was determined as 0.033 mg/mL by HPLC.

### Example 13. Preparing the ophthalmic formulation of the present invention

The materials and proportions used are shown in Table 1, and the preparation procedures were the same as that of Example 1, while the content determination were the same as that of Example 2, to obtain the solution;

The test results of the particle size: the particle size 474.3 nm (98.7%); PdI 0.229; the content by HPLC: 0.089 mg/mL.

The product was stored at 40 °C in dark for 15 days, and the appearance was not obviously changed. The particle size was tested to be 425.2 nm (88.6%), PdI 0.245; and the content was determined as 0.087 mg/mL by HPLC.

### Example 14. Preparing the ophthalmic formulation of the present invention

The materials and proportions used are shown in Table 1, and the preparation procedures and the content determination were the same as that of Example 1, to obtain the solution;

The test results of the particle size: the particle size 136.6 nm (86.3%); PdI 0.542; the content by HPLC: 0.009 mg/mL.

The product was stored at 2-8 °C in dark for 15 days, and the appearance was not obviously changed. The particle size was tested to be 214.2 nm (98.6%), PdI 0.232; and the content was determined as 0.008 mg/mL by HPLC.

### Example 15. Preparing the ophthalmic formulation of the present invention

The materials and proportions used are shown in Table 1, and the preparation procedures were the same as that of Example 1, while the content determination were the same as that of Example 2, to obtain the solution;

The test results of the particle size: the particle size 397.2 nm (90.1%); PdI 0.649; the content by HPLC: 0.015 mg/mL.

The product was stored at 40 °C in dark for 15 days, and the appearance was not obviously changed. The particle size was tested to be 345.6 nm (85.6%), PdI 0.431; and the content was determined as 0.014 mg/mL by HPLC.

### Example 16. Preparing the ophthalmic formulation of the present invention

The materials and proportions used are shown in Table 1, and the preparation procedures and the content determination were the same as that of Example 1, to obtain the solution;

The test results of the particle size: the particle size 13.2 nm (98.3%); PdI 0.235; the content by HPLC: 0.071 mg/mL.

The product was stored at 2-8 °C in dark for 15 days, and the appearance was not obviously changed. The particle size was tested to be 13.8 nm (93.6%), PdI 0.316; and the content was determined as 0.069 mg/mL by HPLC.

### Example 17. Preparing the ophthalmic formulation of the present invention

The materials and proportions used are shown in Table 1, and the preparation procedures and the content determination were the same as that of Example 1, to obtain the solution;

The test results of the particle size: the particle size 527.5 nm (99.4%); PdI 0.240; the content by HPLC: 0.058 mg/mL.

The product was stored at 40 °C in dark for 15 days, and the appearance was not obviously changed. The particle size was tested to be 161.3 nm (100.0%), PdI 0.505; and the content was determined as 0.052 mg/mL by HPLC.

### Comparative example 1

The materials and proportions used are shown in Table 1, and the preparation procedures and the content determination were the same as that of Example 1, to obtain the solution;

The test results of the particle size: the particle size 16.86 nm (69.0%); PdI 1.000; the content by HPLC: 0.051 mg/mL.

The solution was placed overnight at room temperature, and then the precipitation was observed, indicating the stability of the formulation was poor.

### Comparative example 2

The materials and proportions used are shown in Table 1, and the preparation procedures and the content determination were the same as that of Example 1, to obtain the solution containing flocculent suspension;

The test results of the particle size: the particle size 369.8 nm (82.8%); PdI 0.554; the content of the supernatant obtained in the example by HPLC: 0.025 mg/mL.

The solution was placed in dark at 40 °C for 15 days, and then the precipitation of the flocculent suspension was observed. The test results of the particle size: 462.1 nm (80.2%); PdI 0.979; the content of the supernatant obtained in the example by HPLC: 0.027 mg/mL.

### Comparative example 3

The materials and proportions used are shown in Table 1, and the preparation procedures and the content determination were the same as that of Example 1, to obtain the solution containing flocculent suspension;

The test results of the particle size: the particle size 313.7 nm (74.9%); PdI 0.587; the content of the supernatant obtained in the example by HPLC: 0.029 mg/mL.

The solution was placed in dark at 40 °C for 15 days, and then the precipitation of the flocculent suspension was observed. The test results of the particle size: 161.4 nm (100.0%); PdI 0.231; the content of the supernatant obtained in the example by HPLC was 0.023 mg/mL, suggesting the content of active substances was low.

### Comparative example 4

The materials and proportions used are shown in Table 1, and the preparation procedures and the content determination were the same as that of Example 1, to obtain the solution containing flocculent suspension. After being placed at room temperature in a dark place for 15 days, the flocculent suspension aggregated and precipitated.

### Comparative example 5

The materials and proportions used are shown in Table 1, and the preparation procedures and the content determination were the same as that of Example 1, to obtain the solution;

The test results of the particle size: the particle size 292.2 nm (72.6%); PdI 1.000; the content by HPLC: 0.029 mg/mL.

The solution was placed in dark at 40 °C for 15 days, and the appearance was not obviously changed. The test results of the particle size: 188.3 nm (100.0%); PdI 0.217; the content detected by HPLC was 0.026 mg/mL, suggesting the content of active substances was reduced.

The experimental results for absorption in lens of animals: two rats (4 eyes) were used, and then the solution was administered at 20 µL/eye by eye drops. After 1.5 h, the animals were euthanized, and then the lens was quickly collected, in which the content of LAN was determined. Test results: the content of LAN in the lens was 2.00±0.41 (µg/mL), and compared with the blank control eye, it was almost at the same level, indicating that the sample in this comparative sample cannot effectively enter the lenses of the animals.

**Table 1. The amount used in the Examples and the Comparative examples.**

| Exampl e | The amount of excipients and API | | | | | A/B/C/D/E weight ratio | Total volume (mL) |
|---|---|---|---|---|---|---|---|
| | Surfactant (A) | Thickenin g agent 1 (B) | Thickening agent 2 (C) | Co-solvent (D) | Active substance (E) | | |
| 1 | Polysorbat e 80 | HPMC | PVPK12 | Liquid PEG | LAN 6.0 mg | 100/24/24/1000 /1 | 60 |
| 2 | Polysorbat e 80 | HPC | PVPK17 | Liquid PEG | 25-HC 3.0 mg | 25/6/6/100/1 | 30 |
| 3 | Polysorbat e 80 | HPMC | PVP K12 | Liquid PEG | LAN 10.0 mg | 25/6/6/500/1 | 100 |
| 4 | Polysorbat e 80 | HPC | PVP K12 | Propylene glycol | LAN 3.0 mg | 25/6/6/500/1 | 30 |
| 5 | Polysorbat e 20 | HPMC | PVP K30 | Liquid PEG | LAN 3.0 mg | 25/6/6/200/1 | 30 |
| 6 | Polysorbat e 80 | HPMC | PVPK17 | PEG-60 hydrogenate d castor oil | LAN 3.0 mg | 25/6/6/600/1 | 30 |
| 7 | Polysorbat e 40 | HPMC | PVP K60 | Liquid PEG | LAN 3.0 mg | 25/6/6/200/1 | 30 |
| 8 | Polysorbat e 80 | HPMC | PVP K30 | Liquid PEG | LAN 3.0 mg | 25/6/6/500/1 | 30 |
| 9 | Polysorbat e 80 | HPC | PVP K15 | Propylene glycol | LAN 3.0 mg | 200/5/6/2500/1 | 30 |
| 10 | Polysorbat e 20 | HPMC | PVP K90 | Polyoxyethy lene castor oil | LAN 3.0 mg | 125/6/6/600/1 | 30 |
| 11 | Polysorbat e 80 | HPMC | PVP K12 | Glycerol | 25-HC 3.0 mg | 25/6/6/250/1 | 30 |
| 12 | Polysorbat e 80 | HPMC | PVP K60 | Liquid PEG | LAN 1.5 mg | 24/8/6/500/1 | 30 |
| 13 | Polysorbat e 80 | HPMC | PVP K17 | Glycerol | 25-HC 3.0 mg | 250/6/6/1750/1 | 30 |
| 14 | Polysorbat e 80 | HPMC | PVP K15 | Propylene glycol | LAN 0.3 mg | 250/30/20/1250 /1 | 30 |
| 15 | Polysorbat e 80 | HPMC | PVP K90 | Liquid PEG | 25-HC 3.0 mg | 25/6/6/300/1 | 30 |
| 16 | Poloxame r P188 | PEG | PVP K30 | PEG-60 hydrogenate d castor oil | LAN 3.0 mg | 6.7/8/40/600/1 | 30 |
| 17 | Polysorbat e 80 | Carbomer 980 | PVP K12 | Propylene glycol | LAN 3.0 mg | 25/6/6/100/1 | 30 |
| Compar ative example 1 | Polysorbat e 80 | CMC-Na | PVP K12 | Liquid PEG | LAN 6.0 mg | 100/24/24/100/ 1 | 60 |
| Compar ative example 2 | Polysorbat e 80 | NA | N/A | Propylene glycol | LAN 3.0 mg | 250/0/0/1000/1 | 30 |
| Compar ative example 3 | Polysorbat e 80 | N/A | PVP K12 | Liquid PEG | LAN 3.0 mg | 400/0/6/600/1 | 30 |
| Compar ative example 4 | N/A | HPMC | PVP K12 | Glycerol | LAN 9.0 mg | 0/6/6/3000/1 | 30 |
| Compar ative example 5 | Polysorbat e 60 | Xanthan gum | PVP K12 | Liquid PEG | LAN 3.0 mg | 260/6/6/750/1 | 30 |

| | | | | | | | |
|---|---|---|---|---|---|---|---|
| Note: 1. HPMC: Hydroxypropyl methylcellulose; 2. PVP: Povidone; 3. HPC: Hydroxypropyl cellulose; 4. PEG: Polyethylene glycol with an average molecular weight of ≤ 5000 Da; 5. CMC-Na: Carboxymethylcellulose sodium; 6. PEG-60 hydrogenated castor oil: Polyoxyethylene hydrogenated castor oil. | | | | | | | |

From the results of the Examples and the Comparative examples, the eye drop system of the present invention contained at least one surfactant, two thickening agents, and a suitable amount of co-solvents. The prepared products were stable and had a high absorption rate in the lenses of animals;

When the eye drop system of the present invention contained one surfactant, but no or only one thickening agent; alternatively, no surfactant, the stability of the prepared products was relatively poor (see Comparative examples 2, 3, and 4).

If ionic polymers were added to the formulations, the prepared products would precipitate and had poor stability after being left for a short period of time (see Comparative example 1);

If the amount of surfactants exceeded the scope of the present invention, the prepared product had a significant impact on the absorption of animal lenses, which could lead to the inability of the active substances to effectively enter the lens (Comparative example 5).

The beneficial effects of the present invention were demonstrated by reference to Experimental examples in the following.

### Experimental example 1: The absorption test in eyes of SD rats (LAN)

After respiratory anesthesia of six healthy adult SD rats (SPF grade, 180-220 g, all male), 20 µL of the test formulation (Example 1) was separately administered to both eyes of each rat (concentration 0.1 mg/mL). 1.5 hours after eye drops, animals were euthanized, the lens and vitreous body were immediately collected and stored at -80 °C for testing. The contents of LAN in the lens and vitreous body were detected using LC/MS/MS method.

### The animal samples were processed as follows:

After homogenizing the vitreous body samples of animals, to 10 µL of the homogenate, was added 40 µL of 70% methanol, and then the solution was sonicated for 2 min and vortexed for 1 min, followed by adding 175 µL of methanol. The resultant solution was vortexed and mixed for 2 min, and then centrifuged at 12000 rpm and 4 °C for 10 min. The supernatant was collected for analysis of liquid chromatography-tandem mass spectrometry (LC-MS/MS).

After homogenizing the lens sample of animals (adding physiological saline to the lens homogenate at a ratio of 1:4), to 50 µL of the resultant solution, was added 175 µL of methanol. The obtained solution was vortexed and mixed for 2 min, and then centrifuged at 12000 rpm and 4 °C for 10 min. The supernatant was collected for LC-MS/MS analysis.

The LC-MS/MS test conditions were as follows: LC-20AD high-performance liquid chromatography system (SHIMADZU)-API4000 triple quadrupole mass spectrometer (Applied Biosystems), equipped with Fortis Pace C18 5UM 2.1 × 30 mm chromatographic column; column temperature 40 °C; mobile phase methanol:water (95:5); flow rate 0.4 mL/min; injection volume 10 µL; for the mass spectrometer, an Atmospheric Pressure Chemical Ionization (APCI) source was selected. The MS conditions are shown in the table below:

| MS conditions | Sample |
|---|---|
| Ion Source: | Heated Nebulizer |
| Polarity: | Positive |
| NC: | 3 |
| CAD: | 12 |
| CUR: | 20 |
| GS1: | 50 |
| GS2: | 50 |
| TEM: | 600 |
| ihe: | ON |
| Scan Type: | MRM (MRM) |
| MRM: | LAN: |
| | *m*/*z* 409.4/109.3; DP 63; EP 9.6; CE 37; CXP 11 |

The test results are shown in Table 2:

**Table 2. The contents of LAN in the lenses and vitreous bodies of rats after eye drops (Mean±SD).**

| Test formulation | Content of LAN (µg/mL) | |
|---|---|---|
| Time (h) | Lens (n=6) | Vitreous body (n=6) |
| 1.5 | 5.16±1.90 | 0.045±0.009 |

For detection of background values in rats, two SD rats (4 eyes) were used. The animals were euthanized and their lenses were quickly removed. The samples were processed using the same method and the content of LAN was measured. Test results: the content of LAN was 2.23 ± 0.86 (µg/mL). As shown, the ophthalmic formulations of the present invention could effectively deliver LAN through the lens barrier and accumulated in the lens by eye drop administration

The above results indicated that the ophthalmic formulations of the present invention could effectively deliver LAN through the lens barrier by eye drops and accumulated in the lens, with almost no retention in the vitreous body.

### Experimental example 2: The absorption test in eyes of New Zealand rabbits (LAN)

Seven healthy adult New Zealand rabbits (SPF grade, 2-2.5 kg, all males) were included. Six rabbits were selected for eye drop administration of the test substances (Example 3), and all of the rabbits were given 50 µL in a single dose. The remaining one rabbit was not given to both eyes as a background control; 1.5 h after administration, the animals were euthanized to collect the aqueous humor, lens, and vitreous body, and then the contents of LAN in the aqueous humor, lens, and vitreous body were measured. Sample processing and LC/MS/MS detection methods were the same as those in absorption tests of eyes in rats. The test results are shown in Table 3:

**Table 3. The contents of LAN in the lenses, aqueous humor, and vitreous bodies of rabbits after eye drops (Mean±RSD).**

| Sampling time point (hr) | Content of LAN (µg/mL) | | | | | |
|---|---|---|---|---|---|---|
| | Test rabbit eyes | | | Background control rabbit eyes | | |
| | Aqueous humor | Lens | Vitreous body | Aqueous humor | Lens | Vitreous body |
| 0 | - | - | - | BLOQ | 0.76±0.01 | BLOQ |
| 1.5 | BLOQ | 2.72±0.16 | BLOQ | - | - | - |

| | | | | | | |
|---|---|---|---|---|---|---|
| Note: BLOQ: Below the detection limit (LOQ = 0.001 µg/mL), not detected. | | | | | | |

The above results further confirmed that the ophthalmic formulations of the present invention could effectively deliver LAN through the lens barrier by eye drop administration, which was enriched in the lens and not in the vitreous body or aqueous humor.

### Experimental example 3: The absorption test in eyes of SD rats (25-HC)

After respiratory anesthesia of six healthy adult SD rats (SPF grade, 180-220 g, all male), 20 µL of the test formulation (Example 15) was separately administered to both eyes of each rat (concentration 0.1 mg/mL). At each time point, the animal was euthanized, and then the lens and vitreous body were immediately collected to test the content of 25-HC. Due to the fact that the lens of the animal almost did not contain 25-HC, no control eye was set up.

In particular, after respiratory anesthesia of rats, 20 µL of the test formulation (Example 15) was separately administered to both eyes of the animal in each group (concentration 0.1 mg/mL). 1.5 h after administration, three animals were euthanized with carbon dioxide, and then the lenses of both eyes were immediately collected and stored at -80 °C for testing. The sample was treated with an equal volume of silver acetate methanol solution (0.10 mM), while other treatment and testing conditions were the same as that of Experimental example 2.

| Scan Type: | MRM (MRM) |
|---|---|
| MRM: | 25-HC: |
| | *m*/*z* 509.5/124.7; DP 63; EP 9.6; CE 37; CXP 11 |

The test results are shown in Table 4:

**Table 4. The content of 25-hydroxycholesterol (25-HC) in the lenses of rats after eye drops.**

| Time (h) | 25-HC (µg/mL) (Mean±RSD) |
|---|---|
| 1.5 | 0.73±0.19 |

No 25-HC was detected in the samples of the aqueous humor and vitreous body (below detection limit, LCQ = 0.001 µg/mL). The above results confirmed that the ophthalmic formulation of the present invention could effectively deliver 25-HC through the lens barrier and accumulated in the lens by eye drop administration.

### Experimental example 4: The absorption and utilization rate of some formulations according to the present invention in animals

After eye drops, only about 10% of the drug enters the inner eye, while the majority enters the body system through the conjunctiva and nasal cavity (Pei-Xue Ling, edited "Ophthalmic Drugs and Formulations", China Light Industry Press, 2010, P6). Therefore, the utilization rate of the formulation according to the present invention was calculated according to the following standards:
For the examples using rats or rabbits as experimental animals, the total absorption rate is: A% = [(C-C₀)Vₗₑₙₛ/V_{administration}]*100%, and the effective absorption rate is A%/10%.
wherein, C is the concentration of the active substance in the lens after eye drop administration, while C₀ is the concentration of the active substance in the lens of the eye without eye drop administration (µg/mL). Rat lens: diameter = 3.87 mm, Vₗₑₙₛ = 0.03 cm³;
Rabbit lens: diameter = 7.9 mm, Vₗₑₙₛ = 0.258 cm³;

The LAN concentration in Examples 1, 3, and 8 was 0.1 mg/mL, that is, 0.1 µg/µL; dosage of eye drops: rat: V_{administration} = 20 µL *0.1 µg/µL = 2 µg; rabbit: V_{administration} = 50 µL 0. 1 µg/µL = 5 µg

Therefore, the total absorption rates and effective absorption rates of the formulations of Examples 1, 3, and 8, obtained by calculation, are shown in Table 5:

**Table 5. The absorption rates of some formulations according to the present invention in animals.**

| Examples | Animals | 1.5 h after eye drops, LAN concentration increased in the lens. | Total absorption rate | Effective absorption rate |
|---|---|---|---|---|
| 1 | Rat | C-C₀ = 5.16 - 2.23 | [(C-C₀)VₗₑₙₛN_{administration}]*100% | 4.4%/10% |
| | | µg/mL = 2.93 µg/mL | =[(2.93*0.03)/2]*100% | =44% |
| | | | =4.4% | |
| 3 | Rabbit | C-C₀ = 2.72 - 0.76 | [(C-C₀)Vₗₑₙₛ/N_{administration}]*100% | 10.1%/10% |
| | | µg/mL = 1.96 µg/mL | =[(1.96*0.258)/5]*100% | =101% |
| | | | =10.1% | |
| 8 | Rat | C-C₀ = 5.68 - 2.23 | [(C-C₀)Vₗₑₙₛ/N_{administration}]*100% | 5.18%/10% |
| | | µg/mL = 3.45 µg/mL | =[(3.45*0.03)/2]*100% | =51.8% |
| | | | =5.18% | |

As shown, the formulations of the present invention could achieve a very high absorption rate in the lens on the basis of low concentration of active substances, and the effective absorption rate in rabbit lenses could even reach 100%. The utilization rate of the formulation according the present invention was very high. The active substances for treating cataracts could enter the lens by targeted delivery, and thus effectively increase the concentration of oxysterols in the lens, so as to treat cataracts while avoid systemic absorption and toxic side effects.

### Experimental example 5: Observation test on administration in a dog with cataract

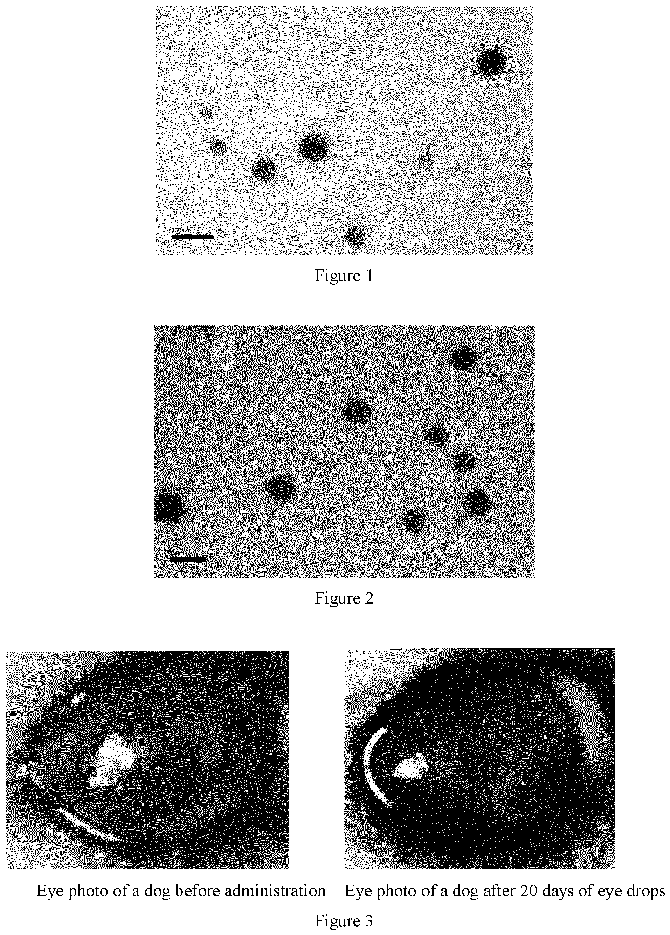

A poodle (aged 15 years, male, weighing 4.0 kg) was kept indoors, regularly and quantitatively fed with dog food and water, and treated entirely as a pet during the experiment. The formulation of Example 3 was administered to the eyes once a day, with approximately 30 µL (one drop). After 20 days of eye drop administration, the range of spontaneous activity for the dog was significantly increased; the partial disappearance of the cataract could be observed after 20 days by photography comparison (Figure 3).

### Experimental example 6: Pharmacodynamic experiment in rats

### 1. Exprimental method A

9-day-old newborn SD rats were selected, and their eye opening was observed daily. When slight eye opening occurred (about 13 days old), sodium selenite solution (Na₂SeO₃, 20 µmol/kg) was given by subcutaneous injection at a volume of 2 mL/kg, and then the same dose of Na₂SeO₃ solution was injected again every other day. After the first injection of Na₂SeO₃ solution, the animals were randomly divided into two groups: a control group and a treatment group. In the control group, both eyes of each mouse were used as the control, and given normal saline (NS) by eye drops, while two eyes of each mouse in the treatment group received the test drug (Example 3) by eye drops. The drug regimen is shown in Table 6:

**Table 6. The drug regimen.**

| | Groups | Animal numbers | Starting point of administration | Eyedrop volume | Times administered by eye drops |
|---|---|---|---|---|---|
| 1 | Model control group | 9 | Administrating after opening eyes | 10 µL NS | 3 times/day |
| 2 | Treatment group | 8 | Administrating after opening eyes | 10 µL test drug | |

### 2. Observation and grading of cataracts

Experimental animals were anesthetized by inhaling isoflurane, and after surface anesthesia with lidocaine, the opacity of the intraocular lens in the rat was observed. The time of cataract formation was recorded and scored according to the following criteria:
Grade 6: Manifested as mature cataracts, involving the entire lens.
Grade 5: Manifested as nuclear opacity that does not involve the lens cortex.
Grade 4: Manifested as partial nuclear opacity.
Grade 3: Manifested as diffuse nuclear opacity with some cortical scattering.
Grade 2: Manifested as mild nuclear opacity, and 2-3 days after injection of selenite, swollen fibers or posterior subscapular opacities produce scattering.
Grade 1: Manifested as initial signs of nuclear turbidity.

### 3. The experimental observation results are shown in Table 7.

**Table 7. The experimental observation results.**

| Eye drop day | D1 | D2 | D4 | D6 |
|---|---|---|---|---|
| Cataract grading | Grade/eye numbers | Grade/eye numbers | Grade/eye numbers | Grade/eye numbers |
| Model control group (18 eyes) | Grade 3/16 eyes | Grade 4/18 eyes | Grade 4/8 eyes | Grade 4/6 eyes |
| | Grade 4/2 eyes | | Grade 5/10 eyes | Grade 5/12 eyes |
| Incidence rate of grade 5 | N/A | N/A | 55.6% | 66.7% |
| | | | | |
| Treatment group (16 eyes) | Grade 3/16 eyes | Grade 3/6 eyes | Grade 4/8 eyes | Grade 4/8 eyes |
| | | Grade 4/10 eyes | Grade 5/8 eyes | Grade 5/8 eyes |
| Incidence rate of grade 5 | N/A | N/A | 50.0% | 50.0% |

Based on the experimental results, on the second day of eye drops, all 18 eyes of animals in the model control group (receiving normal saline by eye drops) developed grade 4 cataracts (100%); while in the treatment group, 10/16 eyes (62.5%) (receiving the test drug by eye drops) developed grade 4 cataracts; on the 6th day, 66.7% of the test eyes in the model group developed grade 5 cataracts, while 50% of the test eyes in the experimental group developed grade 5 cataracts.

### 1. Exprimental method B

9-day-old newborn SD rats were selected, and their eye opening was observed daily. When slight eye opening occurred (about 13 days old), sodium selenite solution (Na₂SeO₃, 20 µmol/kg) was given by subcutaneous injection, with a volume of 2 mL/kg, and then the animals were randomly divided into two groups: a model group and a treatment group. In the model group, both eyes of each mouse were given 10 µL of normal saline (NS) by eye drops at 3 times/day, while two eyes of each mouse in the treatment group received 10 µL of the test drug (Example 3) by eye drops at 3 times/day. The drug regimen is shown in Table 6. The experimental observation results are shown in Table 8.

**Table 8. The experimental observation results.**

| Eye drop day | D0 | D5 | D15 |
|---|---|---|---|
| Cataract grading | On the day of model establishment | Grade/eye numbers | Grade/eye numbers |
| Model group 16 eyes (8 rats) | Grade 0: 16 eyes | Grade 3: 3 eyes | Grade 3: 11 eyes |
| | | Grade 4: 1 eye | Grade 4: 1 eye |
| Treatment group 14 eyes (7 rats) | Grade 0: 14 eyes | Grade 3: 4 eyes | Grade 3: 4 eyes |

On the 15th day of eye drop administration to experimental rats (D15), the incidence rate of grade 3 cataracts in the model group rats was 68.75%, with 1/16 eyes experiencing grade 4 cataracts; the incidence of grade 3 cataracts in the treated group rats was 28.57%, and no eye had grade 4 cataracts.

The above results confirmed that the ophthalmic formulations of the present invention could be administered by eye drops, and the active substances were able to effectively accumulate in the lens, which had the effect of preventing cataracts, delaying the progress of cataracts, and improving cataracts.

In summary, the present invention provided an ophthalmic formulation, which was administered by eye drops for preventing and treating cataracts, and had an excellent stability. Upon eye drop administration, the formulations could allow the active substances to enrich in the lens of an experimental animal, to play the effect of treating and preventing cataracts; moreover, the active substances were not detected in the aqueous humor and the vitreous body, thereby avoiding systematic toxic and side effects. Thus, the present invention provided a solution to the technical problem in the field of ophthalmic drug delivery, that was desired to be solved by those skilled for a long period of time, and had extremely high clinical application values.

## Claims

1. An ophthalmic formulation for eye drop administration, **characterized in that** it is composed of active substances for treating eye diseases and pharmaceutically acceptable carriers or excipients;
the active substances for treating eye diseases are oxysterols, including lanosterol or 25-hydroxycholesterol;
the pharmaceutically acceptable carriers or excipients contain surfactants, thickening agents, cosolvents, and solvents;
the content of oxysterols in the formulation is 0.01-5 mg/mL; and the mass ratio of the surfactant, the thickening agent, the cosolvent, and the oxysterol is (1-300):(1-100):(100-3000):1, with the balance of the formulation being the solvent.

2. The formulation according to claim 1, **characterized in that** the content of oxysterols in the formulation is 0.01-2 mg/mL.

3. The formulation according to claim 2, **characterized in that** the content of oxysterols is 0.05-0.5 mg/mL or 0.01-0.2 mg/mL.

4. The formulation according to claim 1, **characterized in that** the content of oxysterols in the formulation is: 0.01 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.65 mg/mL, 0.7 mg/mL, 0.75 mg/mL, 0.8 mg/mL, 0.85 mg/mL, 0.9 mg/mL, 0.95 mg/mL, 1 mg/mL, 1.5 mg/mL or 2 mg/mL, 2.5 mg/mL, 3 mg/mL, 3.5 mg/mL, 4 mg/mL, 4.5 mg/mL or 5 mg/mL.

5. The formulation according to claim 1, **characterized in that** the mass ratio of the surfactant, the thickening agent, the cosolvent, and the oxysterol is (6.7-250):(11-50):(100-2500):1, preferably (25-200):(11-48):(200-2500):1; and more preferably 25:12:(200-600):1.

6. The formulations according to any one of claims 1 to 5, **characterized in that** the surfactants are non-ionic surfactants.

7. The formulation according to claim 6, **characterized in that** the non-ionic surfactants are polysorbate, poloxamer, or alkyl polyglucoside (APG).

8. The formulations according to any one of claims 1 to 5, **characterized in that** the thickening agent is a combination of at least two of the following polymer compounds, including hydroxypropyl methylcellulose, hydroxypropyl cellulose, methyl cellulose, povidone, carbomer, polyethylene glycol, poloxamer, polyvinyl alcohol, hydroxyethyl cellulose, xanthan gum, hyaluronic acid or its salt, alginic acid or its salt, carboxymethyl cellulose or its salt.

9. The formulation according to claim 8, **characterized in that** the oxysterol is 25-hydroxycholesterol, and the thickening agent is a combination of at least two of the following polymer compounds, including hydroxypropyl methylcellulose, hydroxypropyl cellulose, methyl cellulose, povidone, carbomer, polyethylene glycol, poloxamer, polyvinyl alcohol, hydroxyethyl cellulose, xanthan gum, hyaluronic acid or its salt, alginic acid or its salt, carboxymethyl cellulose or its salt.

10. The formulation according to claim 8, **characterized in that** the oxysterol is lanosterol, and the thickening agent is a combination of at least two of the following polymer compounds, including hydroxypropyl methylcellulose, hydroxypropyl cellulose, methyl cellulose, povidone, carbomer, polyethylene glycol, poloxamer, polyvinyl alcohol, and hydroxyethyl cellulose.

11. The formulations according to any one of claims 8 to 10, **characterized in that** the thickening agent is a combination of two polymer compounds, in which the mass ratio of two polymer compounds is 1:(0.1-10), preferably 1:(0.6-5), and more preferably 1:1.

12. The formulations according to any one of claims 1 to 5, **characterized in that** the solvent in the pharmaceutically acceptable carrier or excipient is a polar solvent, and preferably water.

13. The formulations according to any one of claims 1 to 5, **characterized in that** the co-solvents in the pharmaceutically acceptable carriers or excipients are selected from at least one of liquid polyethylene glycol, propylene glycol, glycerol, polyoxyethylene castor oil, polyoxyethylene hydrogenated castor oil, or castor oil polyoxyethylene ether.

14. The formulations according to any one of claims 1 to 13, **characterized in that** they comprise the following components:
an active substance for treating eye diseases: lanosterol, at a content of 0.01-0.2 mg/mL;
a surfactant: polysorbate or poloxamer, at a content of 6.7-250 times that of lanosterol;
a thickening agent: its content is 11-50 times that of lanosterol, and the thickening agent is a combination of povidone and hydroxypropyl cellulose, wherein the mass ratio of hydroxypropyl cellulose to povidone is 1:(1-1.2); alternatively, the thickening agent is a combination of povidone and hydroxypropyl methylcellulose, wherein the mass ratio of povidone to hydroxypropyl methylcellulose is 1:(1-1.5); alternatively, the thickening agent is a combination of povidone and carbomer, wherein the mass ratio of povidone to carbomer is 1:1; alternatively, the thickening agent is a combination of povidone and polyethylene glycol, wherein the mass ratio of polyethylene glycol to povidone is 1:5;
co-solvent: liquid polyethylene glycol, propylene glycol, glycerol, polyoxyethylene castor oil or castor oil polyoxyethylene ether, at a content of 100-2500 times that of lanosterol; the solvent is water.

15. The formulation according to claim 14, **characterized in that** the content of the surfactant is 25-200 times that of lanosterol; the content of the thickening agent is 11-48 times that of lanosterol; the content of the co-solvent is 200-2500 times that of lanosterol.

16. The formulations according to any one of claims 1 to 13, **characterized in that** they comprise the following components:
an active substance for treating eye diseases: 25-hydroxycholesterol, at a content of 0.1 mg/mL;
a surfactant: polysorbate, at a content of 25-250 times that of 25-hydroxycholesterol;
a thickening agent: its content is 12 times that of 25-hydroxycholesterol, and the thickening agent is a combination of povidone and hydroxypropyl cellulose, wherein the mass ratio of hydroxypropyl cellulose to povidone is 1:1; alternatively, the thickening agent is a combination of povidone and hydroxypropyl methylcellulose, wherein the mass ratio of povidone to hydroxypropyl methylcellulose is 1:1;
co-solvent: liquid polyethylene glycol or glycerol, at a content of 100-1750 times that of 25-hydroxycholesterol; the solvent is water.

17. The formulation according to claim 16, **characterized in that** the content of the surfactant is 25 times that of 25-hydroxycholesterol; the content of the thickening agent is 12 times that of 25-hydroxycholesterol; the content of the co-solvent is 300 times that of 25-hydroxycholesterol.

18. The formulations according to any one of claims 1 to 17, **characterized in that** the pharmaceutically acceptable carriers or excipients in the formulation also comprise any one or more of osmotic pressure regulators, pH regulators, or preservatives;
the osmotic pressure regulators are any one or more of glucose, sodium chloride, potassium chloride, mannitol, sorbitol, sodium citrate, potassium citrate, and glycerol;
the pH regulators are any one or more of hydrochloric acid, sodium hydroxide, acetic acid or its salt, citric acid or its salt, fumaric acid, succinic acid, sorbic acid, phosphoric acid, sodium dihydrogen phosphate, disodium hydrogen phosphate, boric acid, borax, tartaric acid or its salt;
the preservatives are any one or more of sorbic acid, chlorobutanol, sodium chlorite, sodium perborate, quaternary ammonium salts (including benzalkonium chloride, benzalkonium bromide, polyquaternium-1, hexadecyltrimethylammonium bromide), parabens (including methyl hydroxybenzoate, ethyl hydroxybenzoate, propyl hydroxybenzoate), and phenylmercuric nitrate; preferably, the quaternary ammonium salts include benzalkonium chloride, benzalkonium bromide, polyquaternium-1 and/or hexadecyltrimethylammonium bromide, and the parabens include methyl hydroxybenzoate, ethyl hydroxybenzoate, and/or propyl hydroxybenzoate.

19. The formulations according to any one of claims 1 to 18, **characterized in that** the ophthalmic formulations contain nanoparticle structures, which are self-assembled from the carrier or excipient components of the ophthalmic formulation; the nanoparticles comprise active substances for treating eye diseases.

20. The formulation according to claim 19, **characterized in that** the nanoparticles are spherical, with a particle size of 5-900 nm, preferably 5-50 nm and/or 200-700 nm.

21. A method for preparing the formulations according to any one of claims 1 to 20, **characterized in that** it comprises the following steps:
(1) A surfactant and a thickening agent are added to the solvent, and then mixed to obtain a mixed solution;
(2) An active substance for treating eye diseases is added to the mixed solution obtained in step (1), to which a co-solvent is added or not added, and then dispersed and mixed to obtain the initial suspension;
(3) The initial suspension obtained in step (2) is stirred and dispersed and/or homogenized to obtain the formulations.

22. The method according to claim 21, **characterized in that** the dispersion in step (2) is selected from at least one of mechanical stirring and dispersion, magnetic stirring and dispersion, vortex vibration and dispersion, shear dispersion, homogeneous dispersion, grinding and dispersion, and ultrasonic dispersion.

23. The formulations according to any one of claims 1 to 20 for use in the manufacturer of medicaments for the prevention and treatment of lens diseases in humans or animals.

24. The use according to claim 23, **characterized in that** the medicaments are that for preventing and treating cataracts, and preferably that reducing crystallin aggregation and lens opacity.

25. The use according to claim 23, **characterized in that** the medicaments are pharmaceutical formulations for ocular administration, and preferably that for ocular topical administration.
